# EUROPEAN PATENT APPLICATION

(11) **EP 3 196 190 A1**
(43) Date of publication of application: **26.07.2017**
(21) Application number: 15842038.0
(22) Date of filing: 18.09.2015
(51) Int. Cl.: C07D 209/42, A61K 31/404, A61P 3/06, A61P 3/10, A61P 25/28, A61P 35/00, A61P 25/08

(54) **INDOLE DERIVATIVES FOR THE PREVENTION AND/OR TREATMENT OF DIABETES AND ASSOCIATED METABOLIC DISORDERS**

(30) Priority: 19.09.2014 ES 201431364
(71) Applicant: Consejo Superior de Investigaciones Cientificas (CSIC), 28006 Madrid (ES); Centro de Investigación Biomédica en Red (CIBER), 28029 Madrid (ES)
(72) Inventor: CASTRO MORERA, Ana, 28006 Madrid (ES); SANZ BIGORRA, Pascual, 46010 Valencia (ES); VELA RUIZ, Marta, 28006 Madrid (ES); GARCÍA GIMENO, María Adelaida, 28029 Madrid (ES)
(74) Representative: Pons Ariño, Angel
(86) International application number: PCT/ES2015/070677
(87) International publication number: WO 2016/042194

(57) **Abstract**

The invention relates to substituted heterocyclic indole derivatives of Formula (I), which act as activators of the AMP-activated protein kinase (AMPK) and to the use of them for the treatment and prevention of diseases or disorders regulated by AMPK. As a result, these compounds can be used for the treatment of inflammatory, autoimmune, cardiovascular, neurological diseases and cancer.

## Description

### SECTOR OF THE INVENTION

The present invention relates to a series of indole derivative compounds and their use in the treatment of inflammatory, self-immune, cardiovascular, neurological diseases and cancer. The present invention also relates to the pharmaceutical compositions that contain the said compounds. Therefore, the invention belongs to the field of pharmaceutical chemistry.

### STATE OF THE ART

Metabolic syndrome is described as a group of risk factors that include: arterial hypertension, abdominal obesity, high triglyceride levels, low HDL cholesterol levels and fasting hyperglycaemia. This last factor may be considered a characteristic trait of type 2 diabetes. People with metabolic syndrome have a higher probability of developing macro- and micro-vascular complications, which occur in patients with type 2 diabetes, such as arteriosclerosis or other cardiovascular diseases.

Treatments for type 2 diabetes currently exist. Pharmacological treatments include, namely: 1) Inhibition of hepatic glucose production through the use of biguanides such as phenformin or metformin; 2) Use of PPAR agonists (peroxisome proliferator-activated receptors) such as rosiglitazone and troglitazone, as a means of palliating insulin resistance; 3) Increase in the secretion of insulin through sulfonylureas such as tolbutamide, glipizide, etc.; 4) Development of GLP-1 (glucagon-like peptide 1) analogues and derivatives such as exenatide or incretin mimetics such as liraglutide; 5) DPP-4 (dipeptidyl peptidase 4) inhibitors such as esitaglipine or incretin degradation inhibitors. Each of these compounds has its limitations and risks. Thus, phenformin and metformin can induce lactic acidosis, nausea and diarrhea, although metformin has a lower risk in this regard and is widely prescribed for the treatment of type 2 diabetes [Evans J.M. 2005, BMJ, 330:1304-1305]. Currently marketed PPAR agonists show a discrete effect on the reduction of serum glucose and do not significantly improve lipidic metabolism. Sulfonylureas and other insulin liberators can perform their action even with low glucose levels, running the risk of producing hypoglycaemia. Therefore, there is currently still a need to find diabetes treatments that are activated by novel action mechanisms, reinforcing the interest in developing new pharmacological approaches for its treatment and prevention [Shomali M. 2012 Ther Adv Endocrinol Metab 3: 163-173; Tahrani AA., Bailey CJ et al. 2011 Lancet 378: 182-197].

In this context, recent studies have demonstrated that the alteration of cellular energy homeostasis is intimately related to phenomena associated with metabolic syndrome. Specifically, AMP-activated serine/threonine protein kinase (AMPK) appears to be a relevant biological target for these types of pathologies, since it acts as a key sensor of the cellular energy status, coordinating metabolic routes and non-metabolic processes with the objective of balancing the nutrient supply and energy demand levels. AMPK responds to changes in intracellular AMP levels, stimulating the routes associated with ATP generation and/or inhibiting ATP-consuming processes. Consequently, the synthesis of glucose, lipids and proteins, and cellular growth is inhibited, while fatty acid oxidation and glucose recapture are activated [Carling D; Thornton C et al. 2012 Biochem J 445: 11-27; Hardie DG; Ross FA et al. 2012 Nature reviews 13: 251-262; Hardie DG, Ross FA et al. 2012 Chemistry and Biology 19: 1222-1236; Steinberg GR, Kemp BE 2009 Physiological reviews 89: 1025-1078]. This function in the homeostasis of cellular energy confers AMPK an important role in various metabolic disorders, such as type 2 diabetes, obesity and other related disorders. Therefore, compounds capable of regulating energy metabolism, through AMPK modulation, appear to be a promising strategy for preventing and treating these types of diseases [Zhang BB, Zhou G, et al. 2009 Cell Metab 9: 407-416].

Likewise, the action of AMPK on energy metabolism would be responsible for the beneficial effects that the activation of this kinase has on the heart, particularly in the protection against the damages caused by the ischemia-reperfusion process. It has also been observed that AMPK activation decreases cardiovascular hypertrophy and the risk of heart failure, due to which the administration of AMPK-activating compounds would be recommended in these types of pathologies [Zaha VG y Young LH. 2012 Circ Res 111: 800-814].

More recently, the participation of AMPK in metabolic regulation, not only at cellular level but also at the level of whole body energy status, has become more evident. It has been proven that adipocyte-derived hormone leptin triggers AMPK stimulation and, therefore, an increase in the oxidation of fatty acids in skeletal muscles [Minokoshi Y. et al, 2002, Nature, 415, 339]. Adiponectin, another adipocyte-derived hormone that leads to an improved metabolism of carbohydrates and lipids, has been proven to stimulate AMPK in the liver and in skeletal muscles [Yamanauchi T. et al., 2002, Nature Medicine, 8, 1288]. AMPK activation in these circumstances does not seem to be triggered by an increase in AMP cellular levels, but rather by the phosphorylation of the catalytic subunit by one or more upstream kinases.

Based on the aforementioned metabolic consequences of AMPK activation, profound beneficial effects would be expected from the in vivo activation of AMPK. Thus, in the liver, the gene expression of gluconeogenic enzymes would be decreased, thereby reducing hepatic glucose production, and global glucose homeostasis. Furthermore, the direct inhibition and/or reduced expression of the key enzymes in the metabolism of lipids would reduce their synthesis and would also increase the oxidation of fatty acids. This would lead to an improvement resulting from glucose homeostasis and also to an improvement in the action of insulin, due to a reduction in the accumulation of triglycerides in skeletal muscles. Therefore, it would be expected that the combination of these effects on the metabolic syndrome significantly reduces the risk of cardiovascular disease acquisition.

AMPK participates in the regulation of the PI3K/Akt/mTOR route. mTOR is a serine/threonine kinase enzyme that regulates protein synthesis. AMPK negatively regulates the mTOR route at various levels. First, AMPK phosphorylates and activates the tuberous sclerosis protein 2 (TSC2) protein, which is an mTOR inhibitor. Secondly, AMPK phosphorylates and deactivates the raptor protein, a subunit of the mTOR complex, giving rise to the inactivation of the complex. In this way, AMPK activation inhibits cellular growth and protects cells from the apoptosis induced by glucose fasting [Burkewitz K, Zhang Y and Mair W.B. 2014 Cell Metabolism 20: 10-25]. AMPK can also be a therapeutic target for many cancers which have constitutive activation of the PI3K/Akt signalling route. The treatment of different cancer cell lines with AICAR (5-aminoimidazole-4-carboxamide, 1-β-ribofuranoside), an AMP analogue capable of stimulating AMPK, attenuates cellular proliferation both in in vitro and in vivo studies [Rattan R, Giri S, Singh AK, Singh I. 2005 J Biol Chem. 280:39582-39593].

The activation of AMPK by AICAR has proven to reduce FAS and ACC lipogenic enzyme expression, leading to the suppression of the proliferation of cancerous prostrate cells. Many cancerous cells deploy a markedly increased in de novo fatty acid synthesis which correlates with high FAS levels. FAS inhibition suppresses the proliferation of cancerous cells and induces cell death. Therefore, AMPK activation and the inhibition of FAS activity is a clear target for the pharmacological therapy of this type of cancer.

Furthermore, some publications disclose that AICAR is an AMPK activator that exerts an anti-inflammatory effect. AICAR has been observed to attenuate the production of cytokines and pro-inflammatory mediators. AICAR has also been observed to attenuate the progression of experimental self-immune encephalomyelitis, limiting the infiltration of leukocytes through the haematoencephalic barrier, due to which it has been suggested that AMPK activating agents act as anti-inflammatory agents and can maintain therapeutical potential in Krabbe/Twitcher's Disease, which is an inherited neurological disorder [Giri S et al. 2008, J. Neurochem., 105: 1820-1833].

It has also been disclosed that the activation of the AMPK complex plays a neuroprotector role in different neurodegenerative diseases. For example, it has been proven that AMPK activation can exert its neuroprotective effect through the regulation of PGC-1 and UCP2 levels against brain damage induced during status epilepticus [Han et al., 2011, Neurosci Lett 500: 133-138] or through their role in excitotoxicity and apoptosis processes in hippocampal neurons [Ullah et al., 2014, CNS Neurosci Ther 20: 327-338].

### BRIEF DESCRIPTION

The authors of the present invention have found a family of compounds characterised in that they are powerful AMPK enzyme activators. Therefore, the compounds of the invention may prove useful in the treatment or prophylaxis of diseases related to the regulation of said enzyme.

The present invention also relates to the use of a compound of general formula (I) 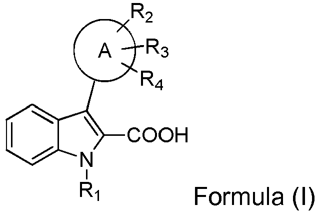 its pharmaceutically acceptable salts, tautomers, pro-drugs, solvates and hydrates where,
- A is selected from between 5- or 6-membered phenyl and heteroaryl containing one heteroatom selected from between nitrogen and oxygen,
- R₁ is selected from among hydrogen, benzyl and 2-(3-hydroxypyridyl)methyl;
- R₂, R₃ y R₄ are independently selected from among hydrogen, alkyl C₁-C₁₈, C₃-C₁₀ cycloalkyl, C₂-C₁₀heterocycloalkyl, C₂-C₁₈alkenyl, hydroxyl, C₁-C₆-O-alkyl, optionally substituted C₇-C₁₂ -O-aralkyl, -NRₐR_{b}, -NCORₐ, -CO₂Rₐ, - CONRₐR_{b}, halogen, -CN, -NO₂, -CORₐ, C₅-C₁₈ aryl and optionally substituted C₄-C₁₈, with the condition that at least one of R₂, R₃ or R₄ is different from hydrogen when A is phenyl;
- Rₐ y R_{b} are selected independently from among hydrogen, C₁-C₆ alkyl, C₃-C₇ cycloalkyl, C₂-C₇ heterocycloalkyl, C₂-C₁₈ alkenyl, C₅-C₁₈ aryl and optionally substituted C₅-C₁₈ heteroaryl, Rₐ and R_{b} can form a C₃-C₇ carbocycle;
to prepare a medicament for treating and/or preventing a disease, disorder or prophylaxis regulated by AMPK enzyme.

Likewise, the present invention also relates to a compound of formula (II) 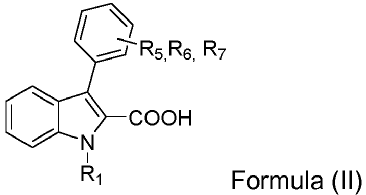 its pharmaceutically acceptable salts, tautomers, pro-drugs, hydrates and solvents where,
- R₁ is selected from among hydrogen, benzyl and 2-(3-hydroxypiridyl)methyl;
- R₅, R₆ and R₇ are independently selected from among hydrogen, C₃-C₁₀ cycloalkyl, C₂-C₁₀ heterocycloalkyl, hydroxyl, optionally substituted C₇-C₁₂ -O-aralkyl, -NRₐR_{b}, -NCORₐ, -CO₂Rₐ, -CONRₐR_{b}, -CN, -NO₂, C₅-C₁₈ aryl and optionally substituted C₄-C₁₈ heteroaryl, with the condition that at least one of R₅, R₆ or R₇ is different from hydrogen;
- Rₐ and R_{b} are independently selected from among hydrogen, C₁-C₆ alkyl, C₃-C₇ cycloalkyl, C₂-C₇ heterocycloalkyl, C₂-C₁₈ alkenyl, C₅-C₁₈ aryl and optionally substituted C₄-C₁₈heteroaryl;

The present invention also relates to a pharmaceutical composition comprising a compound of formula (II) or a pharmaceutically acceptable isomer, pro-drug, salt or solvate thereof and, at least, one pharmaceutically acceptable adjuvant, carrier or excipient.

Additionally, the present invention relates to the use of a compound of formula (II) or a pharmaceutically acceptable isomer, pro-drug, salt or solvate thereof to manufacture amedicament.

### DETAILED DESCRIPTION OF THE INVENTION

In a first aspect, the present invention relates to the use of a compound of general formula (I) 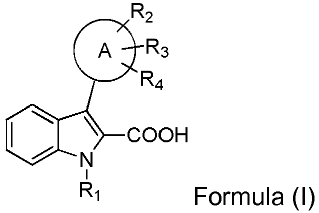 its pharmaceutically acceptable salts, tautomers, pro-drugs, solvates and hydrates where,
- A is selected from between 5- or 6-membered phenyl and heteroaryl containing one heteroatom selected from between nitrogen and oxygen,
- R₁ is selected from among hydrogen, benzyl and 2-(3-hydroxypyridyl)methyl;
- R₂, R₃ and R₄ are independently selected from among hydrogen, C₁-C₁₈ alkyl, C₃-C₁₀ cycloalkyl, C₂-C₁₀ heterocycloalkyl, C₂-C₁₈alkenyl, hydroxyl, C₁-C₆ -O-alkyl, optionally substituted C₇-C₁₂ -O-aralkyl, -NRₐR_{b}, -NCORₐ, - CO₂Rₐ, -CONRₐR_{b}, halogen, -CN, -NO₂, -CORₐ, C₆-C₁₈ aryl and optionally substituted C₄-C₁₈ heteroaryl, with the condition that at least one of R₂, R₃ or R₄ is different from hydrogen when A is phenyl;
- Rₐ and R_{b} are independently selected from among hydrogen, C₁-C₆ alkyl, C₃-C₇ cycloalkyl, C₂-C₇ heterocycloalkyl, C₂-C₁₈ alkenyl, C₅-C₁₈ aryl and optionally substituted C₄-C₁₈ heteroaryl, Rₐ and R_{b} can form a C₃-C₇ carbocycle; to prepare a medicament for treating and/or preventing a disease, disorder or prophylaxis mediated by the AMPK enzyme.

In the present invention, the term "C₁-C₁₈ alkyl" relates to aliphatic, linear or branched chains containing between 1 and 18 carbon atoms, preferably between 1 and 9 carbon atoms, such as for example, but not limited to, methyl, ethyl, n-propyl, i-propyl, n-butyl, terc-butyl, sec-butyl, n-pentyl, n-hexyl, n-heptyl, 1',1'-dimethylheptyl, 1,2-dimethylheptyl or 1',1'-dimethylethyl. While the term "C₁-C₆ alkyl" relates to aliphatic, linear or branched chains containing between 1 and 6 carbon atoms, such as for example, but not limited to, methyl, ethyl, n-propyl, i-propyl, n-butyl, terc-butyl, sec-butyl, n-pentyl or n-hexyl. For both terms, the alkyl group may be optionally substituted by one or more substitutes such as halogen, hydroxyl, C₁-C₆ -O-alkyl, C₁-C₆ -CO-alkyl, -CN, -COOH, C₁-C₆ -COO-alkyl, C₁-C₆ -CONH-alkyl or C₁-C₆ -SO₂-alkyl. Examples of substituted alkyl groups are, but not limited to, 1-(1,3-dithiolane)hexyl, 1-hydroxyethyl or hydroxymethyl, indol-3-ylmethyl, 1,1-dimethyl-5-cyanopentyl, 1,1-cyclopentyloheptane, 1,1dimethyl-heptane, 1,1-di(trifluoromethyl)hexane.

The term "C₂-C₁₈ alkenyl" relates, in the present invention, to carbonated, linear or branched chains having at least one double bond and containing at least between 2 and 18 carbon atoms, preferably between 2 and 9 carbon atoms, such as for example, but not limited to, vinyl, 1-propenyl, 2-propenyl, 1-butenyl, 2-butenyl, 3-butenyl, 1,3-butadienyl, 3-methyl-2-butenyl, 1-hexenyl, 2-hexenyl, 3-hexenyl, 1-dodecenyl or similar. While the term "C₂-C₆ alkenyl" relates to carbonated, linear or branched chains having at least one double bond and containing between 2 and 6 carbon atoms, such as for example, but not limited to, vinyl, 1-propenyl, 2-propenyl, 1-butenyl, 2-butenyl, 3-butenyl, 1,3-butadienyl, 3-methyl-2-butenyl. For both terms, the alkenyl group may optionally be substituted by one or more substitutes such as halogen, hidroxyl, C₁-C₆-O-alkyl, C₁-C₆ -CO-alkyl, -CN, -COOH, C₁-C₆ -COO-alkyl, C₁-C₆ -CONH-alkyl or C₁-C₆-SO₂-alkyl.

The term "C₃-C₁₀ cycloalkyl" relates to a stable carbonated-chain radical that forms a cycle of 3 to 10 carbon atoms, such as for example, but not limited to, cyclopropyl, cyclobutyl, cyclopentyl, cyclohexyl, adamantyl, 1-cyclopentylhexyl. The cycloalkyl group may optionally be substituted by one or more substitutes such as halogen, hydroxyl, C₁-C₆ -O-alkyl, C₁-C₆ -CO-alkyl, - CN, -COOH, C₁-C₆ -COO-alkyl, C₁-C₆ -CONH-alkyl or C₁-C₆ -SO₂-alkyl. Thus, a C₃-C₇ cycloalkyl relates to a stable carbonated-chain radical that forms a cycle of 3 to 7 carbon atoms.

The term "C₂-C₁₀ heterocycloalkyl" relates to a stable 3- to 15-membered ring radical consisting of carbon atoms and between one and five heteroatoms selected from among the group consisting of nitrogen, oxygen and sulphur, preferably a 5- or 6-membered ring containing one or more heteroatoms. Heterocycloalkyl, according to this invention, may be a monocyclic or bicyclic ring system that can include systems of condensed rings and a nitrogen, carbon or sulphur atom in the heterocycloalkyl radical may optionally be oxidised; the nitrogen atom may optionally be quarternised; and the heterocycloalkyl radical may be partially unsaturated. Examples of such heterocycles include, but are not limited to, piperidine, piperazine, pyrrolidine, tetrahydrofuran, tetrahydropyran and morpholine. The heterocycloalkyl group may optionally be substituted by one or more substitutes such as halogen, hydroxyl, C₁-C₆ -O-alkyl, C₁-C₆ -CO-alkyl, -CN, -COOH, C₁-C₆ -COO-alkyl, C₁-C₆ -CONH-alkyl or C₁-C₆ -SO₂-alkyl. Thus, a C₂-C₇ heterocycloalkyl relates to a stable ring radical containing between 2 and 7 carbon atoms.

The term "C₆-C₁₈ aryl" relates, in the present invention, to a stable ring radical containing between 6 and 18 carbon atoms, which may be a monocyclic or multicyclic ring system that may include condensed ring systems. The aryl groups are, for example, but not limited to, phenyl, napthtyl, diphenyl, indenyl, phenantryl or antracyl. Preferably, the aryl group contains between 6 and 8 carbon atoms and, more preferably, the aryl group is a phenyl. The aryl radicals may optionally be substituted by one or more substitutes such as halogen, C₁-C₁₈ alkyl, C₂-C₁₈ alkenyl, C₃-C₇ cycloalkyl, halogen, hydroxyl, C₁-C₆ -O-alkyl, optionally substituted C₇-C₁₁ -O-aralkyl, amine, C₁-C₆ -CO-alkyl, C₃-C₇ -CO-cycloalkyl, -CN, -NO₂, -COOH, C₁-C₆ -COO-alkyl, C₁-C₆ -CONH-alkyl, C₁-C₆ - NCO-alkyl or C₁-C₆ -SO₂-alkyl. Substituted aryl radicals are, for example, but not limited to, 2,4-dichlorophenyl, 1,3-dichlorophenyl, 3,5-difluorophenl and 2-hydroxyphenyl.

The term "C₇-C₁₂ aralkyl" relates, in the present invention, to a stable aliphatic-, linear- or branched-chain radical containing between 1 and 6 carbon atoms each, bonded to a monocyclic aryl containing between 6 and 18 carbon atoms. An example of C₇ aralkyl is benzyl radical.

The term "C₄-C₁₈ heteroaryl" relates to a stable 5- to 21-membered ring radical consisting of carbon atoms and between one and five heteroatoms selected from the group consisting of nitrogen, oxygen and sulphur, preferably a 5- or 6-membered ring containing one or more heteroatoms. Heteroaryl, according to this invention, may be a monocyclic or bicyclic ring system that can include condensed ring systems and the nitrogen atom may optionally be quarternised. Examples of heteroaryl radicals include, but are not limited to, imidazole, pyrrole, pyridine, piperidine, pyrazine, quinoline, indole, thiophene, furan, oxazole and pyrazol.

The term "halogen" relates, in the present invention, to bromine, chlorine, iodine or fluoride.

The term "optionally substituted" or "substituted" includes groups substituted by one to four substitutes, independently selected from among lower alkyl, lower aryl, lower aralkyl, lower alicyclic, hydroxy, lower alkoxy, lower ariloxy, perhaloalkoxy, aralkoxy, heteroaryl, heteroaryloxy, heteroarylalkyl, azide, amine, guanidine, amidine, halogen, lower alkylthio, oxo, acylalkyl, carboxy esters, carboxyl, -carboxamide, nitro, acyloxy, aminoalkyl, alkylaminoaryl, alkylaryl, alkylaminoalkyl, alkoxyaryl, arylamine, aralkylamine, phosphonyl, sulfonyl, -carboxamidoalkylaryl, -carboxamidoaryl, hydroxylalkyl, haloalkyl, alkylaminoalkylcarboxy-, aminocarboxamidoalkyl-, cyano, lower alkoxyalkyl, lower perhaloalkyl, perhaloalkoxy and arylalkyloxyalkyl.

It must be understood that the present invention encompasses all the isomers of the compounds of formula (I), i.e. all the geometric, tautomeric and optical forms, and their mixtures (for example, racemic mixtures). When there are more chiral centres in the compounds of formula (I), the present invention includes, within its scope, all the possible diastereomers, including their mixtures. The different isomeric forms may be separated or resolved therebetween using conventional methods or any given isomer can be obtained using conventional synthetic methods or by means of stereospecific, stereoselective or asymmetric synthesis. The present invention also includes isotope-marked compounds, which are identical to those mentioned in formulas (I) and (II) except that one or more atoms have been replaced with an atom having an atomic mass or mass number different to the atomic mass or mass number usually found in nature. The examples of isotopes that can be incorporated to compounds of the invention include hydrogen, carbon, nitrogen, oxygen, fluoride, iodine and chlorine isotopes, such as ³H, ¹¹C, ¹⁴C, ¹⁸F, ¹²³I and ¹²⁵I.

The scope of the present invention includes compounds of the present invention and pharmaceutically acceptable salts of said compounds containing the aforementioned isotopes and/or other isotopes of other atoms. The isotope-marked compounds of the present invention, for example those to which radioactive isotopes such as ³H or ¹⁴C are incorporated, are useful in assays on drug and/or substrate distribution in tissues. Tritium isotopes are particularly preferred, i.e. ³H, and carbon-14, i.e. ¹⁴C, due to its ease of preparation and detectability. ¹¹C and ¹⁸F isotopes are particularly useful in PET (Positron Emission Tomography), and ¹²⁵I isotopes are particularly useful in SPECT (Single Photon Emission Computerised Tomography), all useful in the formation of images in the brain. Additionally, substitution for heavier isotopes such as deuterium, i.e. ²H, can provide some therapeutic advantages arising from the greater metabolic stability, for example, longer average life in vivo or fewer dosage requirements and, therefore, preferred in some cases. The isotope-marked compounds of formula (I) can generally be prepared following the procedures described in the examples hereunder, substituting a non-isotope-marked reagent for an easily obtainable isotope-marked reagent.

The term "tautomer" or "tautomeric form", as used in the present invention, relates to structural isomers of different energies that are interconvertible via a low-energy barrier. For example, protonic tautomers (also known as prototropic tautomers), which include interconversions via the migration of a proton, such as for example keto-enol or imine-enamine isomerisations. Valencia tautomers include interconversions via the reorganisation of some bonding electrons.

It shall be observed that, for pharmaceutical use, the aforementioned salts shall be physiologically acceptable salts, but other salts can be used, for example, in the preparation of compounds of formula (I) and their physiologically acceptable salts. Pharmaceutically acceptable salts include those described by Berge, Bighley and Monkhouse, J. Pharm. Sci., 1977, 66, 1-19. The expression "pharmaceutically acceptable salts" relates to salts prepared using pharmaceutically acceptable non-toxic bases, including inorganic bases and organic bases. Salts derived from inorganic bases include aluminium, ammonium, calcium, copper, ferric, ferrous, lithium, magnesium, manganic, potassium, sodium, zinc and similar salts. Salts derived from pharmaceutically acceptable non-toxic bases include primary, secondary and tertiary amines, substituted amines including natural substituted amines, cyclic amines and basic ion exchange resins, such as arginine, betaine, caffeine, choline, N,N'-dibenzylethylenediamine, diethylamine, 2-diethylaminoethanol, 2-dimethylaminoethanol, ethanolamine, ethylendiamine, N-etil-morpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine and similar salts. When the compound of the present invention is basic, salts can be prepared from pharmaceutically acceptable non-toxic acids, including inorganic and organic acids. Such acids include acetic, benzenesulfonic, benzoic, canforsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantotenic, phosphoric, succinic, sulfuric, tartaric, ptoluenesulfonic and similar acids.

Preferred examples of pharmaceutically acceptable salts include ammonium, calcium, magnesium, potassium and sodium salts, and those formed from maleic, fumaric, benzoic, ascorbic, pamoic, succinic, hydrochloric, sulfuric, bismethylenesalicylic, methanesulfonic, ethanedisulfonic, propionic, tartaric, salicilyc, citric, gluconic, aspartic, stearic, palmitic, itaconic, glycolic, p-aminebenzoic, glutamic, benzenesulfonic, cyclohexylsulfamic, phosphoric and nitric acids.

Particularly favourite derivatives or pro-drugs are those which increase the bioavailability of the compounds of this invention when such compounds are administered to a patient (for example, making an orally administered compound be more easily absorbed through the blood stream) or that potentiate the release of the original compound in a biological compartment (for example, a tumour) in relation to the original species.

Any compound which is a pro-drug of a compound of formula (I) falls within the scope of the invention. The term "pro-drug" is used in its broadest sense and encompasses derivatives that are converted in vivo in the compounds of the invention. Such derivatives will be evident to the persons skilled in the art and include, depending on the functional groups present in the molecule and without limitation, the following derivatives of the present compounds: esters, amino acid esters, phosphate esters, metal salt sulfonate esters, carbamates and amides.

The compounds of formula (I) may be in crystalline form as free compounds or as solvates and it is intended to include both forms within the scope of the present invention. Solvation methods are generally known in the art. The adequate solvates are pharmaceutically acceptable solvates. In a particular embodiment, the solvate is a hydrate.

The compounds of formula (I) or its salts or solvates are preferably in a pharmaceutically acceptable or substantially pure form. Pharmaceutically acceptable form is understood to be, inter alia, that it has a pharmaceutically acceptable level of purity, excluding normal pharmaceutical additives such as dilutes and carriers, and not including material considered toxic at normal dosage levels. The levels of purity for the main ingredient are preferably greater than 50%, more preferably, greater than 70%, more preferably, greater than 90%. In a preferred embodiment, they are greater than 95% of the compound of formula (I) or of its salts, solvates or pro-drugs.

In a preferred embodiment, the present invention relates to the use of a compound of general formula (I) where, R₁ is selected from between hydrogen and 2-(3-hydroxypyridyl)methyl.

In a preferred embodiment, the present invention relates to the use of a compound of general formula (I) where, R₁ is benzyl or 2-(3-hydroxypyridyl)methyl.

In a preferred embodiment, the present invention relates to the use of a compound of general formula (I) where, A is phenyl or pyridinyl.

In a more preferred embodiment, the present invention relates to the use of a compound of general formula (I) where, A is phenyl.

In a preferred embodiment, the present invention relates to the use of a compound of general formula (I) where, R₄ is selected from among C₁-C₆ -O-alkyl, optionally substituted C₇-C₁₂ -O-aralkyl, -NRₐR_{b}, -NCORₐ, C₂-C₁₀ heterocycloalkyl, C₆-C₁₈aryl and optionally substituted C₄-C₁₈heteroaryl.

In a more preferred embodiment, the present invention relates to the use of a compound of general formula (I) where, R₄ is selected from among -OMe, - OEt, -OBn, -NH₂, -NCOMe, piperidinyl and morpholinyl.

In a more preferred embodiment, the present invention relates to the use of a compound of general formula (I) where, R₃ and R₄ are independently selected from among C₁-C₆ -O-alkyl, optionally substituted C₇-C₁₂ -O-aralkyl, - NRₐR_{b}, -NCORₐ, C₂-C₁₀ heterocycloalkyl, C₆-C₁₈ aryl and optionally substituted C₄-C₁₈heteroaryl.

In an even more preferred embodiment, the present invention relates to the use of a compound of general formula (I) where, R₃ and R₄ are independently selected from among -OMe, -OEt, -OBn, -NH₂, -NCOMe, piperidinyl and morpholinyl.

In a more preferred embodiment, the present invention relates to the use of a compound of general formula (I) where, R₄ is an optionally substituted phenyl.

In an even more preferred embodiment, the present invention relates to the use of a compound of general formula (I) where, R₄ is a phenyl substituted by a hydroxyl group.

In a preferred embodiment, the present invention relates to the use of a compound of general formula (I) where, A is phenyl and R₄ is selected from among C₁-C₆-O-alkyl, optionally substituted C₇-C₁₂-O-aralkyl, -NRₐR_{b}, -NCORₐ, C₂-C₁₀ heterocycloalkyl, C₆-C₁₈ aryl and optionally substituted C₄-C₁₈ heteroaryl.

In a more preferred embodiment, the present invention relates to the use of a compound of general formula (I) where, A is phenyl and R₄ is selected from among -OMe, -OEt, -OBn, -NH₂, -NCOMe, piperidinyl, morpholinyl and optionally substituted phenyl.

In a more preferred embodiment, the present invention relates to the use of a compound of general formula (I) where, A is phenyl and R₃ and R₄ are independently selected from among C₁-C₆ -O-alkyl, optionally substituted C₇-C₁₂ -O-aralkyl, -NRₐR_{b}, -NCORₐ, C₂-C₁₀ heterocycloalkyl, C₆-C₁₈ aryl and optionally substituted C₄-C₁₈heteroaryl.

In a more preferred embodiment, the present invention relates to the use of a compound of general formula (I) where, A is phenyl and R₄ is independently selected from between C₆-C₁₈ aryl and optionally substituted C₄-C₁₈ heteroaryl.

In an even more preferred embodiment, the present invention relates to the use of a compound of general formula (I) where, A is phenyl and R₄ is an optionally substituted phenyl.

In a still more preferred embodiment, the present invention relates to the use of a compound of general formula (I) where, A is phenyl and R₄ is a phenyl substituted by a hydroxyl.

In a preferred embodiment, the compound of general formula (I) is selected from the list comprising:
1-benzyl-3-(2'-hydroxy-[1,1'-biphenyl]-4-yl)-1H-indole-2-carboxylic acid (7)
1-benzyl-3-(4-methoxypyridine-3-yl)-1H-indole-2-carboxylic acid (8)
1-benzyl-3-(4-fluoro-3-methoxyphenyl)-1H-indole-2-carboxylic acid (9)
3-(2'-hydroxy-[1,1'-biphenyl]-4-yl)-1H-indole-2-carboxylic acid (16)
3-(3-(hydroxycarbonyl)phenyl)-1H-indole-2-carboxylic acid (17)
3-(3-benzyloxyphenyl)-1H-indole-2-carboxylic acid (18)
3-(4-fluoro-3-methoxyphenyl)-1H-indole-2-carboxylic acid (19)
3-(furan-2-yl)-1H-indole-2-carboxylic acid (20)
3-(4-methoxypyridine-3-yl)-1H-indols-2-carboxylic acid (21)
3-(pyridine-4-yl)-1H-indole-2-carboxylic acid (22)

In a more preferred embodiment, the compound of general formula (I) is selected from the list comprising:
1 -benzyl-3-(2'-hydroxy-[1,1'-biphenyl]-4-yl)-1 H-indole-2-carboxylic acid (7)
3-(2'-hydroxy-[1,1'-biphenyl]-4-yl)-1H-indole-2-carboxylic acid (16)
3-(3-(hydroxycarbonyl)phenyl)-1H-indole-2-carboxylic acid (17)
3-(3-benzyloxyphenyl)-1H-indole-2-carboxylic acid (18)

In an even more preferred embodiment, the compound of general formula (I) is selected from the list comprising:
1-benxyl-3-(2'-hydroxy-[1,1'-biphenyl]-4-il)-1H-indole-2-carboxylic acid (7)
3-(2'-hydroxy-[1,1'-biphenyl]-4-yl)-1H-indole-2-carboxylic acid (16)

According to this description, any of the previously defined compounds, i.e. compounds of general formula (I), can be equally referred to herein as "compound or compounds of the invention."

In a second aspect, the present invention relates to a compound of general formula (II), 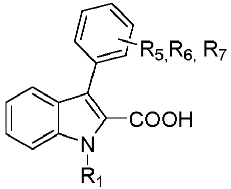 its pharmaceutically acceptable salts, tautomers, pro-drugs, hydrates where,
- R₁ is selected from among hydrogen, benzyl and 2-(3-hydroxypyridyl)methyl;
- R₅, R₆ and R₇ are independently selected from among hydrogen, C₃-C₁₀ cycloalkyl, C₂-C₁₀ heterocycloalkyl, hidroxyl, optionally substituted C₇-C₁₂ -O-aralkyl, -NRₐR_{b}, -NCORₐ, -CO₂Rₐ, -CONRₐR_{b}, -CN, -NO₂, C₅-C₁₈ aryl and optionally substituted C₄-C₁₈ heteroaryl, with the condition that at least R₅, R₆ or R₇ is different to hydrogen;
- Rₐ and R_{b} are independently selected from among hydrogen, C₁-C₆ alkyl, C₃-C₇ cycloalkyl, C₂-C₇ heterocycloalkyl, C₂-C₁₈ alkenyl, C₅-C₁₈ aryl and optionally substituted C₄-C₁₈ heteroaryl, Rₐ and R_{b} can form C₃-C₇ carbocycle;

In a preferred embodiment, the present invention relates to a compound of general formula (II) where, R₁ is selected from between hydrogen and 2-(3-hydroxypyridyl)methyl.

In a preferred embodiment, the present invention relates to a compound of general formula (II) where, R₁ is selected from between benzyl and 2-(3-hydroxypyridyl)methyl.

In a preferred embodiment, the present invention relates to a compound of general formula (II) where, R₇ is selected from among C₃-C₁₀ -cycloalkyl, C₂-C₁₀ heterocycloalkyl, optionally substituted C₇-C₁₂ -O-aralkyl, -NRₐR_{b}, -NCORₐ, - CO₂Rₐ, -CONRₐR_{b}, -CN, -NO₂ where, Rₐ and R_{b} are defined as above.

In a more preferred embodiment, the present invention relates to a compound of general formula (II) where, R₇ is selected from among -OBn, - COOH, -NH₂, -NCOMe, piperidinyl and morpholinyl.

In a preferred embodiment, the present invention relates to a compound of general formula (II) where, R₆ and R₇ are independently selected from among - C₃-C₁₀ cycloalkyl, C₂-C₁₀ heterocycloalkyl, optionally substituted C₇-C₁₂ -O-aralkyl, -NRₐR_{b}, -NCORₐ, -CO₂Rₐ, -CONRₐR_{b}, -CN, -NO₂ where, Rₐ and R_{b} are defined as above.

In a more preferred embodiment, the present invention relates to a compound of general formula (II) where, R₆ and R₇ are independently selected from among -OBn, -COOH, -NH₂, -NCOMe, piperidinyl and morphonyl.

In a preferred embodiment, the compound of general formula (II) is selected from the list comprising:
1-benzyl-3-(2'-hydroxy-[1,1'-biphenyl]-4-yl)-1H-indole-2-carboxylic acid (7)
3-(2'-hydroxy-[1,1'-biphenyl]-4-yl)-1H-indole-2-carboxylic acid (16)
3-(3-(hydroxycarbonyl)phenyl)-1H-indole-2-carboxylic acid (17)
3-(3-benzyloxyphenyl)-1H-indole-2-carboxylic acid (18)

In a preferred embodiment, the present invention relates to a compound of general formula (III), 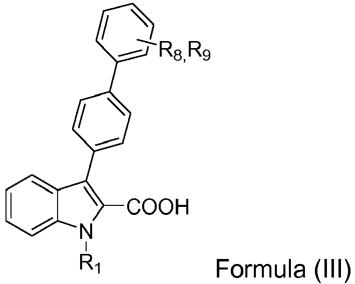 its pharmaceutically acceptable salts, tautomers, pro-drugs, hydrates and solvates where,
- R₁ is selected from among hydrogen, benzyl and 2-(3-hydroxypyridyl)methyl;
- R₈ and R₉ are independently selected from among hydrogen, halogen, hydroxyl, optionally substituted C₇-C₁₂-O-aralkyl, -NR_{c}R_{d}, -NCOR_{c}, -CO₂R_{c}, - CONR_{c}R_{d}, -CN and -NO₂,
with the condition that R₉ is not hydrogen;
- R_{c} and R_{d} are independently selected from among hydrogen, C₁-C₆ alkyl and C₃-C₇ cycloalkyl, R_{c} and R_{d} can form a C₃-C₇ carbocycle.

In a preferred embodiment, the present invention relates to a compound of general formula (III) where, R₁ is selected from between hydrogen and 2-(3-hydroxypyridyl)methyl.

In a preferred embodiment, the present invention relates to a compound of general formula (III) where, R₁ is selected from between benzyl and 2-(3-hydroxypyridyl)methyl.

In a preferred embodiment, the present invention relates to a compound of general formula (III) where, R₉ is selected from among halogen, hydroxyl, - NR_{c}R_{d}, -NCOR_{c}, -CO₂R_{c}, -CONR_{c}R_{d}, -CN and -NO₂ where, R_{c} and R_{d} are independently selected from among hydrogen, C₁-C₆ alkyl and C₃-C₇ cycloalkyl, where R_{c} and R_{d} can form a C₃-C₇ carbocycle.

In a more preferred embodiment, the present invention relates to a compound of general formula (III) where, R₉ is selected from among hydroxyl, - NR_{c}R_{d}, -NCOR_{c}, -CONR_{c}R_{d}, -CN and -NO₂ where, R_{c} and R_{d} are independently selected from among hydrogen, C₁-C₆ alkyl and C₃-C₇ cycloalkyl where, R_{c} and R_{d} can form a C₃-C₇carbocycle.

In an even more preferred embodiment, the present invention relates to a compound of general formula (III) where, R₉ is selected from between hydroxyl and amine (-NH₂).

In a preferred embodiment, the present invention relates to a compound of general formula (III) where, R₈ and R₉ are independently selected from among halogen, hydroxyl, -NR_{c}R_{d}, -NCOR_{c}, -CO₂R_{c}, -CONR_{c}R_{d}, -CN and -NO₂ where, R_{c} and R_{d} are independently selected from among hydrogen, C₁-C₆ alkyl and C₃-C₇ cycloalkyl where, R_{c} and R_{d} can form a C₃-C₇carbocycle.

In an even more preferred embodiment, the present invention relates to a compound of general formula (III) where, R₈ and R₉ are selected from among hydroxyl, -NR_{c}R_{d}, -NCOR_{c}, -CONR_{c}R_{d}, -CN and -NO₂ where, R_{c} and R_{d} are independently selected from among hydrogen, C₁-C₆ alkyl and C₃-C₇ cycloalkyl where, R_{c} and R_{d} can form a C₃-C₇ carbocycle.

In a more preferred embodiment, the compound of general formula (III) is selected from the list comprising:
1 -benzyl-3-(2'-hydroxy-[1,1'-biphenyl]-4-yl)-1H-indole-2-carboxylic acid (7)
3-(2'-hydroxy-[1,1 '-biphenyl]-4-yl)-1H-indole-2-carboxylic acid (16)

In a third aspect, the invention relates to a pharmaceutical composition comprising the compound of formula (II), as described above, and at least one excipient, adjuvant and/or a pharmaceutically acceptable carrier. Additionally, the pharmaceutical composition is envisaged to contain another main ingredient.

Pharmaceutical compositions containing a therapeutically effective amount of a compound of formula (II), its pharmaceutically acceptable isomers, pro-drugs, salts or solvates thereof, together with the pharmaceutically acceptable carriers, constitute an additional aspect of the present invention. It relates to a pharmaceutical composition comprising at least one pharmaceutically acceptable carrier and a therapeutically effective amount of at least one compound of the invention. Hereinafter, said pharmaceutical composition can equally be referred to as "pharmaceutical composition of the invention."

The term "carrier" relates to a dilute, adjuvant or excipient with which the main ingredient is administered. Such pharmaceutical carriers may be sterile liquids, such as water and oils, including those derived from oil, animal, vegetable or synthetic, such as peanut oil, soybean oil, mineral oil, sesame seed oil and similar. Water or aqueous saline solutions and aqueous dextrose and glycerol solutions are preferably used as carriers, particularly for injectable solutions. Appropriate pharmaceutical carriers are described in "Remington's Pharmaceutical Sciences" by E. W. Martin, 1995. Preferably, the carriers of the invention are approved by the regulatory agency of a state government or a federal government, or are enumerated in the United States Pharmacopoeia, in the European Pharmacopoeia or other pharmacopoeia recognised in general for use in animals and, more particularly, in humans.

The therapeutically effective amount of the compound of the invention and the pharmaceutically acceptable isomers, pro-drugs, salts or solvates thereof that must be administered (also referred to herein as therapeutically amount effective thereof), in addition to their dosage for treating a pathological condition with said compounds, will depend on a number of factors, including age, the condition of the patient, the severity of the disease, administration route and frequency, the modular compound to be used, etc.

The pharmaceutical compounds and compositions of this invention can be used on their own or jointly with other drugs to provide combined therapy. The other drugs can form part of the same pharmaceutical composition or be provided as a separate pharmaceutical composition, to be administered simultaneously or at different intervals. Examples of pharmaceutical compositions include any solid composition (tablets, pills, capsules, granules, etc.) or liquid composition (solutions, suspensions or emulsions) for oral, topical or parenteral administration.

The present invention also relates to the compounds of formula (II), as previously defined, to manufacture a medicament.

A further aspect of the present invention relates to the use of the compounds of formula (I) or of a pharmaceutical composition containing at least one compound of formula (II), as previously described, or a pharmaceutically acceptable tautomer, pro-drug or solvate thereof to manufacture a drug or pharmaceutical composition for the treatment and/or prevention of inflammatory, self-immune, cardiovascular, neurological diseases, and cancer, where the AMPK enzyme is involved, such as for example, but not limited to, type 2 diabetes, obesity, dyslipidemia, hypertension, insulin resistance, hypertriglyceridemia, epilepsy, Krabbe/Twitcher's Disease, Alzheimer's Disease, Parkinson's Disease and Huntington's Disease.

The compounds of general formula (I) are AMPK activators and therefore said compounds can be used in the treatment and/or prevention of diseases in which this enzyme is involved.

In another aspect, the invention relates to the use of a compound of formula (I) or of a pharmaceutical composition containing at least one compound of formula (II), as previously described, or a pharmaceutically acceptable tautomer, pro-drug, salt or solvate thereof, to manufacture a medicamentfor the treatment of cardiovascular, inflammatory, self-immune and neurological diseases, metabolic syndrome and cancer.

In a preferred embodiment, the present invention relates to the use of a compound of formula (I) or of a pharmaceutical composition containing at least one compound of formula (II), as previously described, or a pharmaceutically acceptable tautomer, pro-drug, salt or solvate thereof, to manufacture a medicament for the treatment of type 2 diabetes, obesity, dyslipidemia, hypertension, hyperglycemia, hypertriglycerimidemia and insulin resistance.

In a preferred embodiment, the present invention relates to the use of a compound of formula (I) or of a pharmaceutical composition containing at least one compound of formula (II), as previously described, or a pharmaceutically acceptable tautomer, pro-drug, salt or solvate thereof, to manufacture a medicament for the treatment of type 1 diabetes.

In a preferred embodiment, the present invention relates to the use of a compound of formula (I) or of a pharmaceutical composition containing at least one compound of formula (II), as previously described, or a pharmaceutically acceptable tautomer, pro-drug, salt or solvate thereof, to manufacture a medicament for the treatment of epilepsy.

In a preferred embodiment, the present invention relates to the use of a compound of formula (I) or of a pharmaceutical composition containing at least one compound of formula (II), as previously described, or a pharmaceutically acceptable tautomer, pro-drug, salt or solvate thereof, to manufacture a medicament for the treatment of Krabbe/Twitcher's Disease.

In a preferred embodiment, the present invention relates to the use of a compound of formula (I) or of a pharmaceutical composition containing at least one compound of formula (II), as previously described, or a pharmaceutically acceptable tautomer, pro-drug, salt or solvate thereof, to manufacture a medicament for the treatment of Alzheimer's Disease, Parkinson's Disease and Huntington's Disease.

In a preferred embodiment, the present invention relates to the use of a compound of formula (I) or of a pharmaceutical composition containing at least one compound of formula (II), as previously described, or a pharmaceutically acceptable tautomer, pro-drug, salt or solvate thereof, to manufacture a medicament for the treatment of prostate and breast cancer.

According to the present description, the use of a compound of the invention or of a pharmaceutical composition to manufacture a medicament or, alternatively, its use as a medicament, for the treatment of a disorder or disease that can be potentially controlled by means of AMPK activation, can obviously be understood as a method for treating such disorder or disease, which comprises the administration of a therapeutically effective amount of said compound or pharmaceutical composition of the invention to a patient. In other words, the present invention also relates to a method for treating a disorder or disease (preferably selected from among metabolic syndrome and inflammatory, self-immune, cardiovascular and neurological diseases, and cancer and, more preferably, type 1 and 2 diabetes, hyperglycemia, obesity, inflammation, epilepsy, prostate cancer, breast cancer and Krabbe/Twitcher's Disease, Alzheimer's Disease, Parkinson's Disease and Huntington's Disease), which comprises administering a therapeutically effective amount of the compound of the invention or a pharmaceutical composition of the invention comprising the compound of the invention in a therapeutically effective amount to a patient.

Another aspect of the invention relates to a method for obtaining the compounds of the invention according to the following reaction scheme. 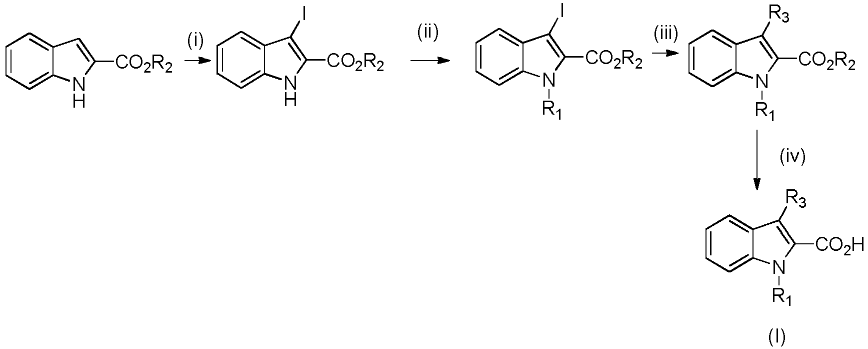 where R₂ is ethoxyl and R₁ and R₃ are previously defined. In step (i), the iodation of the indole derivative takes place; in step (ii), the alkylation of heterocyclic nitrogen; in step (iii), the C-C coupling reaction; and, in step (iv), the hydrolysis of the ester group.

Throughout the description and in the claims, the word "comprises" and its variants are not intended to exclude other technical characteristics, additives, components or steps. For the persons skilled in the art, other objects, advantages and characteristics of the invention shall be inferred partially from the description and partially from the practice of the invention. The following examples are provided by way of illustration and are not intended to be limiting of the present invention.

### DESCRIPTION OF THE FIGURES

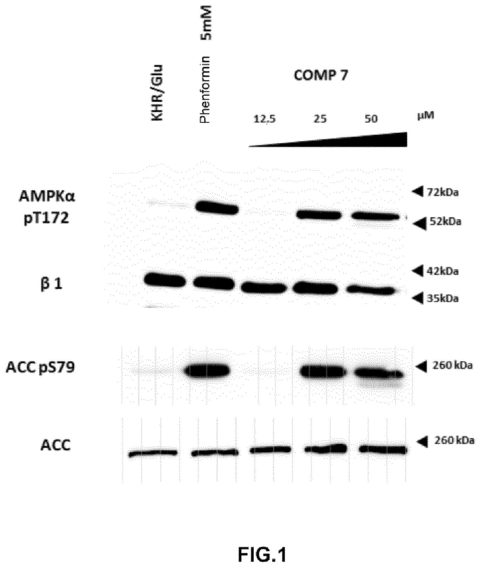

**Fig. 1** Shows the activation of the AMPK of compound **7.** Example of the Western Blot analysis that shows that compound **7** activates AMPK in a dose-dependent manner, this effect is confirmed by the increase in phosphorylation, which is also dependent upon the dose, of its ACC substrate, in a range of concentrations comprised between 12.5 and 50 µM. The HEK293 cells are treated with the amounts of compound indicated in the figure for 1 hour. The lysed cells are analysed by Western Blott using anti-phosphoThr172 AMPK alpha, anti-AMPKb1 (used as a loading control), anti-phosphoSer79ACC and anti-ACC (used as a loading control). The molecular weight markers are indicated on the right of the figure.

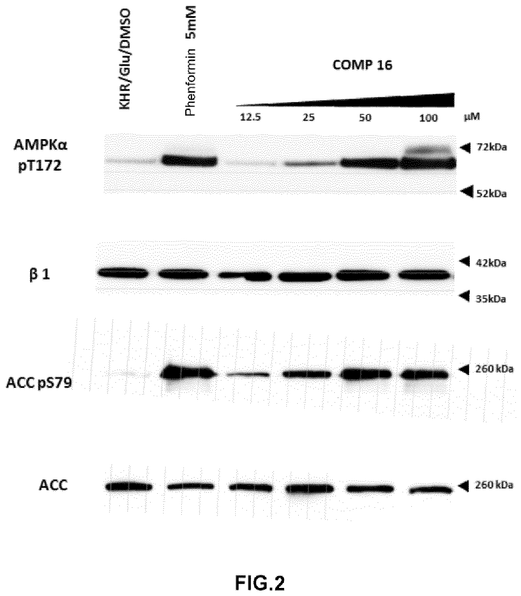

**Fig. 2** Shows the effect of AMPK activation of compound **16.** Example of the Western Blot analysis that shows that compound **16** activates AMPK in a dose-dependent manner, this effect is confirmed by the increase in phosphorylation, which is also dose-dependent, on its ACC substrate, in a range of concentrations comprised between 12.5 and 100 µM. The HEK293 cells are treated with the amounts of compound indicated in the figure for 1 hour. The lysed cells are analysed by Western Blot using anti-phosphoThr172 AMPK alpha, anti-AMPKb1 (used as a loading control), anti-phosphoSer79ACC and anti-ACC (used as a loading control). The molecular weight markers are indicated on the right of the figure.

### EXAMPLES AND EMBODIMENT OF THE INVENTION

The invention is illustrated below by the assays conducted by the inventors, which demonstrate the effectiveness of the compounds of the invention.

### Example 1

### 1.1. Synthesis of compounds 7 - 9 and 16 - 22

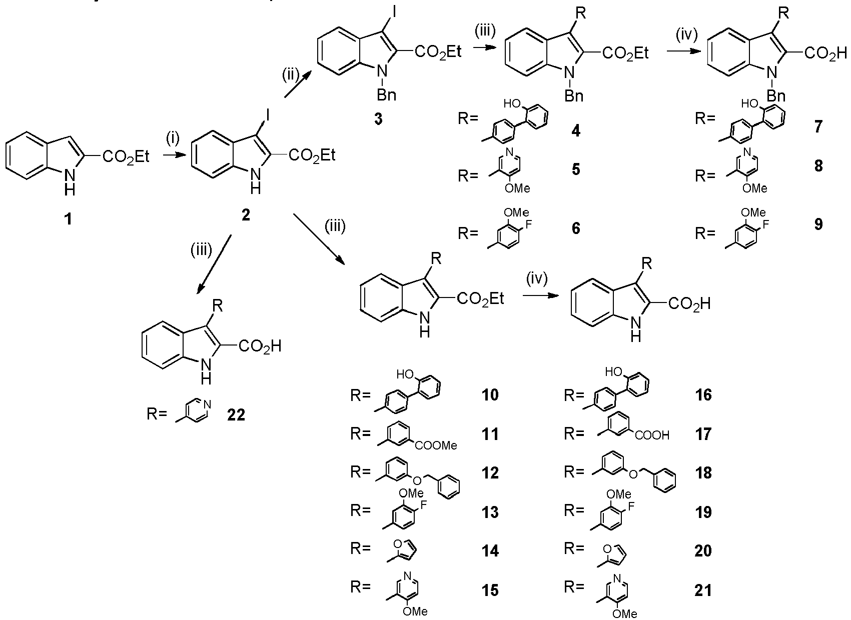

Scheme 1: Where (i) is N-iodosuccinimide, DMF, 0°C, 1h; (ii) NaH, DMF, BrBn, t.a., 2h; (iii) Dioxane:toluene:ethanol:water (10:1:3:6), R-B(OH)₂, Pd(dppf)Cl₂, K₂CO₃, 85°C, 12h; (iv) EtOH:H₂O (2:1), KOH, 100°C, 1h.

### 3-iodo-1H-indole-2-ethyl carboxylate (2)

A solution of 1 H-indole 2-ethyl carboxylate (1.89 g, 10 mmol) in DMF (15 ml) is slowly added to a solution containing NIS (2.25 g, 12 mmol) in DMF (20 ml) at 0°C. The mixture is stirred at room temperature for 1 hour. Next, a solution of sodium thiosulfate at 10% (5ml) and water (10 ml) are added. stirring at room temperature for another hour. During that time, a precipitate appears that is collected by filtration. 2.9 g (96%) of a white solid are obtained. **MS** (ES, positive mode): m/z 315 (96%) (M+1)⁺. **¹H NMR** (DMSO-d₆) δ 12.25 (s, 1 H), 7.78 - 6.88 (m, 4H), 4.38 (q, *J* = 7.1 Hz, 2H), 1.39 (t, *J* = 7.1 Hz, 3H).

### 1-benzyl-3-iodo-1H-indole-2-ethyl carboxylate (3)

NaH (26.4 mg, 1.1 mmol) is added to a solution of iodised derivative 2 (315 mg, 1 mmol) in DMF (3 ml) at 0°C. Next, benzyl bromide (223 mg, 1.3 mmol) is slowly added. The reaction mixture is stirred at room temperature for 2 hours Next, water (10 ml) is added to the mixture and is extracted with AcOEt (2x15 ml). The organic layer is dried over anhydrous Na₂SO₄, filtered and evaporated. The crude obtained is purified by column chromatography (AcOEt:Hex 1:10). 315 mg (78%) of a white solid are obtained. **MS** (ES, positive mode): m/z 405 (95%) **(M+1)⁺. ¹H NMR** (CDCl₃) δ 7.53 (d, *J* = 8.0 Hz, 1H), 7.36 - 7.10 (m, 5H), 6.94 (dd, *J* = 6.8, 1.5 Hz, 2H), 5.73 (s, 3H), 4.30 (q, *J* = 7.1 Hz, 3H), 1.31 (t, *J* = 7.1 Hz, 5H). **¹³C NMR** (CDCl₃) δ 13.2, 48.0, 60.3, 66.8, 109.9, 120.7, 123.0, 125.1, 125.3, 126.2, 127.4, 127.6, 129.4, 136.8, 137.8, 160.1.

### 1-benzyl-3-(2'-hydroxy-[1,1'-biphenyl]-4-yl)-1H-indole-2-ethyl carboxylate (4)

4'-(4,4,5,5-tetramethyl-1,3,2-dioxoborolan-2-yl) biphenyl-2-ol (255 mg, 0.86 mmol), Pd(dppf)Cl₂ (26 mg, 0.036 mmol) and, lastly, an aqueous solution (5 ml) of K₂CO₃ (398 mg, 2.88 mmol) are added to a solution of the iodised benzyl derivative **3** (290 mg, 0.72 mmol) in a solvent mixture of 1,4-dioxane:toluene:ethanol:water 10:1:3:6 (20 ml). The mixture is stirred at 85°C for 3 hours in a nitrogen atmosphere. Once the reaction has concluded, the solvent is evaporated at reduced pressure. The residue is dissolved in water (10 ml) and extracted with AcOEt (3 x 10 ml). The organic layer is dried over anhydrous sodium sulfate, filtered and evaporated. The crude is purified by column chromatography (CH₂Cl₂: MeOH 50:1). 305 mg (95%) are obtained as a beige solid. **MS** (ES, positive mode): m/z 447 (95%) (M+1)⁺. **¹H NMR** (DMSO-*d*₆) δ 9.59 (s, 1 H), 8.05 - 6.45 (m, 17H), 5.79 (s, 2H), 4.12 (q, *J* = 6.5 Hz, 2H), 1.00 (t, *J* = 7.1, 6.5 Hz, 3H). **¹³C NMR** (DMSO-d₆) δ 13.8, 47.8, 55.3, 61.0, 111.7, 116.5, 119.9, 121.3, 121.5, 123.8, 125.0, 125.7, 126.2, 126.7, 127.5, 127.8, 128.8, 128.9, 129.0, 130.0, 130.6, 132.3, 137.5, 138.1, 138.7, 154.8, 162.2.

### 1-benzyl-3-(4-methoxypyridine-3-yl)-1H-indole-2-ethyl carboxylate (5)

(4-methoxypyridine-3-yl)boronic acid (136 mg, 0.9 mmol), Pd(dppf)Cl₂ (28 mg, 0.04 mmol) and, lastly, an aqueous solution (5 ml) of K₂CO₃ (409 mg, 2.96 mmol) are added to a solution of iodised derivative **3** (300 mg, 0.74 mmol) in a solvent mixture of 1,4-dioxane:toluene:ethanol:water 10:1:3:6 (20 ml). The mixture is stirred at 85°C for 2 hours in a nitrogen atmosphere. Once the reaction has concluded, the solvent is evaporated under reduced pressure. The crude obtained is purified by column chromatography (Hexane:Ethyl Acetate 1:1). 40 mg (14%) are obtained as a white solid. **MS** (ES, positive mode): m/z 386 (97%) (M+1)⁺. **¹H NMR (DMSO-*d*₆)** δ 8.23-6.92 (m, 12H), 5.86 (s, 2H), 4.11 (c, *J* = 7.1 Hz, 2H), 3.92 (s, 3H), 1.15 (t, *J* = 7.1 Hz, 3H).

### 1-benzyl-3-(4-fluoro-3-methoxyphenyl)-1H-indole-2-ethyl carboxylate (6)

(4-fluoro-3-methoxyphenyl)boronic acid (204 mg, 1.2 mmol), Pd(dppf)Cl₂ (36 mg, 0.05 mmol) and, lastly, an aqueous solution (5 ml) of K₂CO₃ (553 mg, 4 mmol) are added to a solution of iodised derivative 3 (405 mg, 1 mmol) in a solvent mixture of 1,4-dioxane:toluene:ethanol:water 10:1:3:6 (20 ml). The mixture is stirred at 85°C for 2 hours in a nitrogen atmosphere. Once the reaction has concluded, the solvent is evaporated under reduced pressure. The crude obtained is purified by column chromatography (Hexane:Ethyl Acetate 4:1). 145 mg (36%) are obtained as a white solid. **MS** (ES, positive mode): m/z 403 (94%) **(M+1)⁺. ¹H NMR (DMSO-*d*₆)** δ 7.36 (m, 12H), 5.88 (s, 2H), 4.16 (c, *J* = 7.1 Hz, 2H), 3.93 (s, 3H), 1.05 (t, *J* = 7.1 Hz, 3H).

### 1-benzyl-3-(2'-hydroxy-[1,1'-biphenyl]-4-il)-1H-indole-2-carboxylic acid (7)

KOH (75 mg, 1.34 mmol) is added to a solution of indole **4** (200 mg, 0.45 mmol) in a solvent mixture of ethanol:water 4:1 (20 ml). The reaction mixture is heated at 100°C for 3 hours. The reaction is treated with 1 M of HCl solution (20 ml) and the precipitate is collected by filtration. 125 mg (66%) of a white solid are obtained. **MS** (ES, positive mode): m/z 419 (95%) (M+1)⁺. **¹H NMR** (DMSO-*d*₆) δ 13.11 (s, 1 H), 9.64 (s, 1 H), 8.27 - 6.53 (m, 16H), 5.86 (s, 2H). **¹³C NMR** (DMSO-d₆) δ 47.6, 111.6, 116.5, 119.8, 121.3, 122.9, 125.3, 126.4, 126.8, 127.4, 127.7, 128.9, 130.0, 130.6, 132.7, 137.3, 137.9, 149.4, 154.8, 163.8.

### 1-benzyl-3-(4-methoxypyridine-3-yl)-1H-indole-2-carboxylic acid (8)

KOH (103 mg, 1.55 mmol) is added to a solution of indole 5 (200 mg, 0.52 mmol) in a solvent mixture of ethanol:water 3:2 (20 ml). The reaction mixture is heated at 100°C for 2 hours. The reaction volume is reduced to half and is treated with 1 M of HCl solution (20 ml). The precipitate is collected by filtration. 70 mg (37%) of a white solid are obtained. **MS** (ES, positive mode): m/z 358 (90%) **(M+1)⁺. ¹H NMR** (DMSO-d₆) δ 8.24-6.95 (m, 12H), 5.89 (s, 2H), 3.93 (s, 3H).

### 1-benzyl-3-(4-fluoro-3-methoxyphenyl)-1H-indole-2-carboxylic acid (9)

KOH (49 mg, 0.74 mmol) is added to a solution of indole **6** (100 mg, 0.25 mmol) in a solvent mixture of ethanol:water 3:2 (20 ml). The reaction mixture is heated at 100°C for 2 hours. The reaction volume is reduced by half and is treated with 1 M of HCl solution (20 ml). The precipitate is collected by filtration. 70 mg (74%) of a white solid are obtained. **MS** (ES, positive mode): m/z 375 (93%) (M+1)⁺. **¹H NMR** (DMSO-d₆) δ 7.32 (m, 12H), 5.84 (s, 2H), 3.91 (s, 3H).

### 3-(2'-hydroxy-[1,1'-biphenyl]-4-yl)-1H-indole-2-ethyl carboxylate (10)

4'-(4,4,5,5-tetramethyl-1,3,2-dioxoborolan-2-yl) biphenyl-2-ol (98 mg, 0.33 mmol), Pd(dppf)Cl₂ (10 mg, 0.01 mmol) and, lastly, an aqueous solution (5 ml) of K₂CO₃ (150 mg, 1.08 mmol) are added to a solution of iodised derivative **2** (87 mg, 0.27 mmol) in a solvent mixture of 1,4-dioxane:toluene:ethanol:water 10:1:3:6 (20 ml). The mixture is stirred at 85°C all night in a nitrogen atmosphere. Once the reaction has concluded, the solvent is evaporated at reduce pressure. The residue is dissolved in water (10 ml) and extracted with AcOEt (3 x 10 ml). The organic layer is dried over anhydrous sodium sulfate, filtered and evaporated under reduce pressure. The crude obtained is purified by column chromatography (CH₂Cl₂:MeOH 20:1). 56 mg (60%) are obtained as a beige solid. **MS** (ES, positive mode): m/z 357 (96%) (M+1)⁺. **¹H NMR** (DMSO-*d*₆) δ 11.96 (s, 1H), 9.63(s, 1 H), 8.18 - 6.05 (m, 12H), 4.30 (d, J = 7.1 Hz, 2H), 1.28 (t, J = 7.1 Hz, 3H). **¹³C NMR** (DMSO-d₆) δ 14.4, 60.7, 113.1, 116.5, 119.9, 120.9, 121.1, 122.8, 123.1, 125.5, 127.2, 127.9, 128.8, 130.4, 130.7, 132.1, 136.6, 137.5, 154.8, 161.7.

### 3-(3-(ethoxicarbonyl)phenyl)-1H-indole-2-ethyl carboxylate (11)

3-(methoxycarbonyl)phenyl-boronic acid (108 mg, 0.6 mmol), Pd(dppf)Cl₂ (19 mg, 0.025 mmol) and, lastly, an aqueous solution (5 ml) of K₂CO₃ (275 mg, 2 mmol) are added to a solution of iodised derivative **2** (157 mg, 0.5 mmol) in a solvent mixture of 1,4-dioxane:toluene:ethanol:water 10:1:3:6 (20 ml). The mixture is stirred at 85°C for 2 hours in a nitrogen atmosphere. Once the reaction has concluded, the solvent is evaporated under reduced pressure. The crude obtained is purified by column chromatography (Hexane:Ethyl Acetate 1:1). 120 mg (70%) are obtained as a white solid. **MS** (ES, positive mode): m/z 323 (98%) **(M+1)⁺. ¹H NMR (DMSO-*d*₆)** δ 12.04 (s, 1 H), 8.08-7.12 (m, 8H), 4.22 (q, *J* = 7.1 Hz, 2H), 3.90 (s, 3H), 1.16 (t, *J* = 7.1 Hz, 3H). **¹³C NMR (DMSO-d₆)** δ 166.6, 161.5, 136.5, 135.6, 134.4, 131.4, 129.6, 128.7, 127.9, 126.9, 125.6, 123.4, 121.4, 121.1, 120.5, 113.1, 60.8, 52.5, 14.2.

### 3-(3-benzyloxyphenyl)-1H-indole-2-ethyl carboxylate (12)

(3-benzyloxyphenyl)boronic acid (102 mg, 0.57 mmol), Pd(dppf)Cl₂ (18 mg, 0.02 mmol) and, lastly, an aqueous solution (5 ml) of K₂CO₃ (265 mg, 1.92 mmol) are added to a solution of iodised derivative **2** (150 mg, 0.48 mmol) in the solvent mixture of 1,4-dioxane:toluene:ethanol:water 10:1:3:6 (20 ml). The mixture is stirred at 85°C for 2 hours in a nitrogen atmosphere. Once the reaction has concluded, the crude obtained is purified by column chromatography (CH₂Cl₂:MeOH 50:1). 135 mg (77%) are obtained as a white solid. **MS** (ES, positive mode): m/z 371 (94%) (M+1)⁺. **¹H NMR** (DMSO-d₆) δ 11.91 (s, 1 H), 7.55 - 6.97 (m, 13H), 5.16 (s, 2H), 4.23 (q, *J* = 7.1 Hz, 3H), 1.19 (t, *J* = 7.1 Hz, 3H). **¹³C NMR** (DMSO-d₆) δ 161.75, 157.91, 137.32, 136.27, 134.91, 129.41, 128.90, 128.23, 127.74, 126.84, 125.73, 123.42, 122.87, 122.60, 121.12, 120.79, 117.01, 113.98, 112.92, 69.48, 60.96, 14.14.

### 3-(4-methoxypyridine-3-yl)-1H-indole-2-ethyl carboxylate (13)

(4-fluoro-3-methoxyphenyl)boronic acid (204 mg, 1.2 mmol), Pd(dppf)Cl2 (36 mg, 0.05 mmol) and, lastly, an aqueous solution (5 ml) of K₂CO₃ (553 mg, 4 mmol) are added to a solution of iodised derivative **2** (315 mg, 1 mmol) in the solvent mixture of 1,4-dioxane:toluene:ethanol:water 10:1:3:6 (20 ml). The mixture is stirred at 85°C for 12 hours in a nitrogen atmosphere. Once the reaction has concluded, the solvent is evaporated under reduced pressure. The crude obtained is purified by column chromatography (DCM: MeOH 50:1). 300 mg (95%) are obtained as a white solid. **MS** (ES, positive mode): m/z 313 (93%) (M+1)⁺. **¹H NMR (DMSO-*d*₆)** δ 11.91 (s, 1H), 7.61- 7.24 (m, 7H), 4.26 (c, *J* = 6.9 Hz, 2H), 3.90 (s, 3H), 1.23 (t, *J* = 7.1, 6.6 Hz, 3H).

### 3-(furan-2-yl)-1H-indole-2-ethyl carboxylate (14)

3-(methoxycarbonyl)phenyl-boronic acid (135 mg, 1.2 mmol), Pd(dppf)Cl₂ (37 mg, 0.05 mmol) and, lastly, an aqueous solution (5 ml) of K₂CO₃ (552 mg, 4 mmol) are added to a solution of iodised derivative **2** (315 mg, 1 mmol) in the solvent mixture of 1,4-dioxane:toluene:ethanol:water 10:1:3:6 (20 ml). The mixture is stirred at 85°C for 2 hours in a nitrogen atmosphere. Once the reaction has concluded, the solvent is evaporated at low pressure. The crude obtained is purified by column chromatography (Hexane:Ethyl Acetate 3:2). 240 mg (94%) are obtained as a white solid. **MS** (ES, positive mode): m/z 255 (99%) (M+1)⁺. **¹H NMR (DMSO-d₆)** δ 8.24 - 6.50 (m, 7H), 4.35 (c, *J* = 7.1 Hz, 2H), 1.33 (t, *J =* 7.1 Hz, 3H).

### 3-(4-methoxypyridine-3-yl)-1H-indole-2-ethyl carboxylate (15)

(4-methoxypyridine-3-yl)boronic acid (183 mg, 1.2 mmol), Pd(dppf)Cl2 (36 mg, 0.05 mmol) and, lastly, an aqueous solution (5 ml) of K₂CO₃ (553 mg, 4 mmol) are added to a solution of iodised derivative **2** (315 mg, 1 mmol) in the solvent mixture of 1,4-dioxane:toluene:ethanol:water 10:1:3:6 (20 ml). The mixture is stirred at 85°C for 2 hours in a nitrogen atmosphere. Once the reaction has concluded, the solvent is evaporated under reduced pressure. The crude obtained is purified by column chromatography (Hexane:Ethyl Acetate 5:1). 245 mg (83%) are obtained as a white solid. **MS** (ES, positive mode): m/z 296 (98%) (M+1)⁺. **¹H NMR (DMSO-*d*₆)** 11.98 (s, 1 H), 8.28-6.91 (m, 7H), 4.25 (c, *J* = 7.1 Hz, 2H), 3.92 (s, 3H), 1.21 (t, *J* = 7.1 Hz, 3H).

### 3-(2'-hydroxy-[1,1'-biphenyl]-4-yl)-1H-indole-2-carboxylic acid (16)

KOH (94 mg, 1.68 mmol) is added to a solution of indole 10 (200 mg, 0.56 mmol) in the solvent mixture ethanol:water 2:1 (20 ml). The reaction mixture is heated at 100°C for 1 hour. The reaction is treated with 1 M of HCl solution (20 ml) and the precipitate is collected by filtration. 90 mg (50%) are obtained of a white solid. **MS** (ES, positive mode): m/z 329 (99%) (M+1)⁺. **¹H NMR** (DMSO-d₆) δ 11.82 (s, 1 H), 8.05 - 6.48 (m, 12H). **¹³C NMR** (DMSO-d₆) δ 112.9, 116.5, 119.8, 120.6, 120.9, 122.2, 123.9, 125.1, 127.4, 127.9, 128.8, 130.4, 130.6, 132.4, 136.4, 137.2, 154.8, 163.2.

### 3-(3-(hydroxycarbonyl)phenyl)-1H-indole-2-carboxylic acid (17)

KOH (59 mg, 0.90 mmol) is added to a solution of indole **11** (100 mg, 0.30 mmol) in the solvent mixture of ethanol:water 2:1 (20 ml). The reaction mixture is heated at 100°C for 2 hours. The reaction volume is reduced by half and treated with 1 M of HCl solution (20 ml). The precipitate is collected by filtration. 24 mg (99%) of a white solid are obtained. **MS** (ES, positive mode): m/z 281 (100%) (M+1)⁺. **¹H NMR** (DMSO-d₆) δ 12.95 (s, 1H), 11.91 (s, 1H), 7.68-7.09 (m, 8H). **¹³C NMR (DMSO-*d*₆)** δ 167.75, 163.00, 136.31, 135.24, 134.55, 131.57, 130.72, 128.48, 127.94, 127.13, 125.23, 124.19, 121.12, 120.90, 120.46, 113.05.

### 3-(3-benzyloxyphenyl)-1H-indols-2-carboxylic acid (18)

KOH (51 mg, 0.77 mmol) dissolved in water is slowly added to a solution of indole **12** (95 mg, 0.26 mmol) in ethanol (20 ml). The reaction mixture is heated at 100°C for 3 hours. The reaction mixture is evaporated until dry, treated with 1 M of HCl solution (20 ml) and the precipitate is collected by filtration. After washing with ethyl ether, 18 mg (21%) of a white solid are obtained. **MS** (ES, positive mode): m/z 343 (98%) (M+1)⁺. **¹H NMR** (DMSO-d₆) δ 12.87 (s, 1H), 11.81 (s, 1H), 7.49- 6.95 (m, 13H), 5.15 (s, 2H). **¹³C NMR (DMSO-*d*₆)** δ 163.12, 158.15, 137.61, 136.25, 135.52, 129.09, 128.81, 128.11, 127.94, 127.26, 125.06, 123.92, 123.51, 121.98, 120.86, 120.64, 117.18, 113.66, 112.88, 69.53.

### 3-(4-fluoro-3-methoxyphenyl)-1H-indole-2-carboxylic acid (19)

KOH (97 mg, 1.72 mmol) is added to a solution of indole **13** (180 mg, 0.57 mmol) in a solvent mixture of ethanol:water 6:4 (20 ml). The reaction mixture is heated at 100°C for 1 hour. The reaction volume is reduced by half and treated with 1 M of HCl solution (20 ml), and removed with CH₂Cl₂ (50 ml). The organic layer is dried with Na₂SO₄, filtered and the solvent removed at low pressure. 130 mg (80%) of a white solid are obtained. **MS** (ES, positive mode): m/z 285 (95%) (M+1)⁺. **¹H NMR** (DMSO-d₆) δ12.90 (s, 1 H), 11.81 (s, 1 H), 7.60 - 7.02 (m, 7H), 3.90 (s, 3H).

### 3-(furan-2-yl)-1H-indole-2-carboxylic acid (20)

KOH (138 mg, 2.05 mmol) is added to a solution of indole **14** (175 mg, 0.68 mmol) in a solvent mixture of ethanol:water 3:1 (20 ml). The reaction mixture is heated at 100°C for 2 hours. The reaction volume is reduced by half and treated with 1 M of HCl solution (20 ml). The precipitate is collected by filtration. 70 mg (45%) of a white solid are obtained. **MS** (ES, positive mode): m/z 227 (98%) (M+1)⁺. **¹H NMR** (DMSO-d₆) δ 13.18 (s, 1 H), 11.91 (s, 1 H), 8.08-6.92 (m, 7H).

### 3-(4-methoxypyridine-3-yl)-1H-indole-2-carboxylic acid (21)

KOH (127 mg, 1.92 mmol) is added to a solution of indole **15** (190 mg, 0.64 mmol) in a solvent mixture of ethanol:water 15:3 (20 ml). The reaction mixture is heated at 100°C for 3 hours. The reaction volume is reduced by half and treated with 1 M of HCl solution (20 ml). The precipitate is collected by filtration. 120 mg (68%) of a white solid are obtained. **MS** (ES, positive mode): m/z 268 (97%) (M+1)⁺. **¹H NMR (DMSO-*d*₆)** δ 11.91 (s, 1 H), 8.30 (s, 1 H), 7.49-6.93 (m, 7H), 3.87 (s, 3H).

### 3-(pyridine-4-yl)-1H-indole-2-carboxylic acid (22)

(4pyridinyl)boronic acid (141 mg, 1.1 mmol), Pd(dppf)Cl₂ (35 mg, 0.05 mmol) and, lastly, an aqueous solution (5 ml) of K₂CO₃ (525 mg, 3.8 mmol) are added to a solution of iodised derivative **2** (300 mg, 0.95 mmol) in a solvent mixture of 1,4-dioxane:toluene:ethanol 10:1:3 (20 ml). The mixture is stirred at 85°C for 16 hours in a nitrogen atmosphere. Once the reaction has concluded, the solvent is evaporated at low pressure and removed with AcOEt/H₂O (2x20 ml). The aqueous layer is acidulated with 1 M of HCl solution (20 ml), giving rise to a precipitate that is isolated by filtration. 200 mg (88%) are obtained as a beige solid. **MS** (ES, positive mode): m/z 238 (99%) (M+1)⁺. **¹H NMR (DMSO-*d*₆**) δ 12.57 (s, 1 H), 9.10 - 8.73 (m, 2H), 8.37 - 8.05 (m, 2H), 7.75 - 7.15 (m, 4H).

### Example 2

AMPK activation measurements in cultured cells by means of compounds **7** and **16.**

**Cell line and treatments.** The cell line that was used in the treatments with the different assayed reagents was HEK293T (human embryonic kidney cells). Cells were grown in DMEM (Dulbecco's Modified Eagle's Medium) with 25 mM of glucose supplemented with 10% of inactivated fetal bovine serum, 2 mM glutamine, 100 units/ml of penicillin and 100 µg/ml of streptomycin, in a humid atmosphere at 37°C with 5% of CO₂. Cells were grown on 60 mm (p.60) plates to obtain 70-80% of confluence. Cells were washed in Krebs Ringer buffer (KRB: NaCl 12.5 mM, CaCl₂ 15 mM, KH₂PO₄ 0.5 mM, KCI 3 mM, NaHCO₃ 2,5 mM, MgSO₄ 0.5 mM, HEPES 10 mM pH 7.4, 95:5 O₂/CO₂) tempered at 37°C and subsequently treated for 1 hour at 37°C in a culture oven, adding the adequate quantities of **7** and **16** (5 mM stock in DMSO) dissolved in KRB/25 mM glucose to reach the final concentrations indicated in the figures. Phenformin 5 mM was used as an activation control.

**Preparation of HEK293T cell extracts.** After the corresponding treatments, the supernatant was removed and cells were quickly-frozen in liquid N₂. The plates were processed one by one and kept in ice , firstly adding the cold lysis buffer. The composition of the buffer was as follows: Tris 10 mM pH 7.4, EDTA 15 mM pH 8.0, NaF 50 mM, Na₄P₂O₇ 15 mM, sacarose 0.6 M, 2-Mercaptoethanol 15 mM, a mixture of protease inhibitors without EDTA (Roche) and 1 mM PMSF. Cells were collected in a lysis buffer with the help of a scraper and were lysed, passing each sample through 24 Gx5/8" syringes four times. A small amount was reserved to measure the quantity of protein using the Bradford protein assay and loading buffer for electrophoresis was added to the rest and boiled for 5 min maintaining the samples at -20°C until their use. The concentration of proteins was determined through the Bradford method using the Bio-Rad Bradford Protein Assay Reagent (BioRad).

**Protein analysis by Western Blot.** The protein extracts were analysed using SDS-PAGE in gels with 8% or 10% acrylamide and 1.5 mm thick. 30 µg of protein were loaded and transferred to a PVDF (Millipore) membrane for 1.5 hours at 100 V. Blockage was performed using 5% of skimmed milk in TBS-T for 1 hour at room temperature. Immunodetection was performed by incubating the primary antibody all night at 4°C. After three 10-minute washes at room temperature using TBS-T, they were incubated with their corresponding secondary antibody conjugated to HRP. After the three 10-minute washes at room temperature using TBS-T, the membranes were developed using ECL plus (Pierce) and processed using a FUJI LAS 3000 (Fujifilm) unit. The antibodies used were: anti-pAMPKaThr172, anti-AMPKβ1/β2, anti-ACC and anti-pACCser79 from the company Cell Signaling Technology (Danvers, MA, USA) diluted 1:1000, and the HRP goat anti-rabbit secondary antibody from the company Santa Cruz Technology at a dilution of 1:5000 or 1:10000. The detection of bands with the anti-pAMPKαThr172 and anti-pACCser79 antibodies (AMPK substrate) with respect to their respective loading controls (anti-AMPKβ1/β2 and anti-ACC) was taken as an indicative of AMPK activation.

### Results. Description of the dose-response effect of compounds 7 and 16.

The activation status of the AMPK complex is correlated with the levels of the phosphorylated form of the AMPKα pT172 catalytic subunit. As can be observed in the first panel of the corresponding figures, the treatment of the HEK293 cells with increasing doses of products **7** or **16** produced an increase in endogenous levels of AMPKα pT172, being an indication of the increased state of activation of the AMPK complex. The activation reached in some cases was similar to that achieved with phenformin, a compound with renowned AMPK activation capacity.

The second panel shows the analysis of the endogenous levels of AMPKβ1 that were included as a loading control to demonstrate that the increase in AMPKα pT172 levels were not due to an increase in the total levels of the enzyme complex.

The third panel shows the state of phosphorylation of the acetyl CoA carboxylase (ACC) enzyme. This enzyme is a substrate of the AMPK kinase protein, due to which an increase in AMPK activity produces an increase in the state of phosphorylation of this substrate (ACC pS79). As can be observed, the state of phosphorylation of ACC increases in parallel to the AMPKα pT172 levels.

The fourth panel indicates the total levels of ACC as a loading control, to validate that the change in ACC pS79 levels are due to the phosphorylation of the protein and not to an increase in the total levels thereof.

All these data indicate that compounds **7** and **16** are capable of inducing the activation of the AMPK complex in vivo at the indicated doses.

## Claims

1. Use of a compound of formula (I), 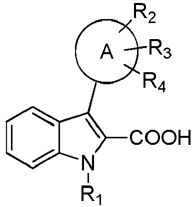 its pharmaceutically acceptable salts, solvates and hydrates where,
- A is selected from between 5- or 6-membered phenyl or heteroaryl containing a heteroatom selected from between nitrogen and oxygen,
- R₁ is selected from among hydrogen, benzyl and 2-(3-hydroxypyridyl)methyl;
- R₂, R₃ and R₄ are independently selected from among hydrogen, C₁-C₁₈ alkyl, C₃-C₁₀ cycloalkyl, C₂-C₁₀ heterocycloalkyl, C₂-C₁₈ alkenyl, hydroxyl, C₁-C₆-O-alkyl, optionally substituted C₇-C₁₂-O-aralkyl, -NRₐR_{b}, -NCORₐ, - CO₂Rₐ, -CONRₐR_{b}, halogen, -CN, -NO₂, -CORₐ, C₆-C₁₈ aryl and optionally substituted C₄-C₁₈ heteroaryl,
with the condition that at least one of R₂, R₃ or R₄ is different from hydrogen when A is phenyl; and
- Rₐ and R_{b} are independently selected from among hydrogen, C₁-C₆ alkyl, C₃-C₇ cycloalkyl, C₂-C₇ heterocycloalkyl, C₂-C₁₈ alkenyl, C₅-C₁₈ aryl and optionally substituted C₄-C₁₈ heteroaryl, Rₐ and R_{b} can form a C₃-C₇ carbocycle;
to prepare a medicament for the treatment and/or prevention of a disease, disorder or prophylaxis mediated by the AMPK enzyme.

2. The use, according to the preceding claim, where A is selected from between phenyl and pyridinyl.

3. The use, according to claim 1, where A is phenyl and R₄ is selected from among C₁-C₆ -O-alkyl, optionally substituted C₇-C₁₂-O-aralkyl, -NRₐR_{b}, -NCORₐ, C₂-C₁₀ heterocycloalkyl, C₆-C₁₈ aryl and optionally substituted C₄-C₁₈ heteroaryl.

4. The use, according to the preceding claim, where R₄ is selected from among -OMe, -OEt, -OBn, -NH₂, -NCOMe, piperidinyl, morpholinyl and optionally substituted phenyl.

5. The use of a compound of formula (I), according to claim 1, selected from the list comprising:
1-benzyl-3-(2'-hydroxy-[1,1'-biphenyl]-4-yl)-1H-indole-2-carboxylic acid (7)
1-benzyl-3-(4-methoxypyridine-3-yl)-1H-indole-2-carboxylic acid (8)
1-benzyl-3-(4-fluoro-3-methoxyphenyl)-1H-indole-2-carboxylic acid (9)
3-(2'-hydroxy-[1,1'-biphenyl]-4-yl)-1H-indole-2-carboxylic acid (16)
3-(3-(hydroxycarbonyl)phenyl)-1H-indole-2-carboxylic acid (17)
3-(3-benzyloxyphenyl)-1 H-indole-2-carboxylic acid (18)
3-(4-fluoro-3-methoxyphenyl)-1H-indole-2-carboxylic acid (19)
3-(furan-2-yl)-1H-indole-2-carboxylic acid (20)
3-(4-methoxypyridine-3-yl)-1H-indole-2-carboxylic acid (21)
3-(pyridine-4-yl)-1H-indole-2-carboxylic acid (22).

6. The use, according to any of claims 1 to 5, where the AMPK enzyme-mediated disease is selected from among type 2 diabetes, obesity, dyslipidemia, hypertension, hyperglycemia, hypertriglycerimidemia and insulin resistance.

7. The use, according to any of claims 1 to 5, where the AMPK enzyme-mediated disease is selected from among Alzheimer's Disease, Parkinson's Disease and Huntington's Disease.

8. The use, according to any of claims 1 to 5, where the AMPK enzyme-mediated disease is prostate cancer and breast cancer.

9. The use, according to any of claims 1 to 5, where the AMPK enzyme-mediated disease is epilepsy.

10. A compound of formula (II), 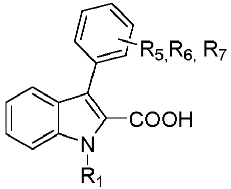 its pharmaceutically acceptable salts, hydrates and solvates where,
- R₁ is selected from among hydrogen, benzyl and 2-(3-hydroxypyridyl)methyl;
- R₅, R₆ and R₇ are independently selected from among hydrogen, C₃-C₁₀ cycloalkyl, C₂-C₁₀ heterocyclolkyl, hydroxyl, optionally substituted C₇-C₁₂ - O-aralkyl, -NRₐR_{b}, -NCORₐ, -CO₂Rₐ, -CONRₐR_{b}, -CN, -NO₂, C₅-C₁₈ aryl and optionally substituted C₄-C₁₈heteroaryl,
with the condition that at least one of R₅, R₆ or R₇ is different from hydrogen;
- Rₐ and R_{b} are independently selected from among hydrogen, C₁-C₆ alkyl, C₃-C₇ cycloalkyl, C₂-C₇ heterocycloalkyl, C₂-C₁₈alkenyl, C₅-C₁₈ aryl and optionally substituted C₄-C₁₈heteroaryl; and
Rₐ and R_{b} can form a C₃-C₇ carbocycle.

11. A compound, according to the preceding claim, where R₇ is selected from among C₃-C₁₀ -cycloalkyl, C₂-C₁₀ heterocycloalkyl, optionally substituted C₇-C₁₂-O-aralkyl, -NRₐR_{b}, -NCORₐ, -CO₂Rₐ, -CONRₐR_{b}, -CN and -NO₂.

12. The compound, according to claim 10, which is selected from the list comprising:
1-benzyl-3-(2'-hydroxy-[1,1'-biphenyl]-4-yl)-1H-indole-2-carboxylic acid (7)
3-(2'-hydroxy-[1,1'-biphenyl]-4-yl)-1H-indole-2-carboxylic acid (16)
3-(3-(hydroxycarbonyl)phenyl)-1H-indole-2-carboxylic acid (17)
3-(3-benzyloxyphenyl)-1H-indole-2-carboxylic acid (18).

13. The compound of formula (III), according to claim 10, 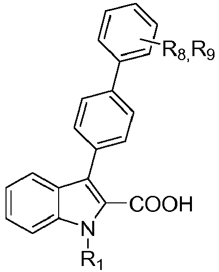 its pharmaceutically acceptable salts, hydrates and solvates where,
- R₁ is selected from among hydrogen, benzyl and 2-(3-hydroxypyridyl)methyl;
- R₈ and R₉ are independently selected from among hydrogen, halogen, hydroxyl, optionally substituted C₇-C₁₂ -O-aralkyl, -NR_{c}R_{d}, -NCOR_{c}, - CO₂R_{c} -CONR_{c}R_{d}, -CN and -NO₂,
with the condition that R₉ is not hydrogen;
- R_{c} and R_{d} are independently selected from among hydrogen, C₁-C₆ alkyl and C₃-C₇cycloalkyl; and
R_{c} and R_{d} can form a C₃-C₇ carbocycle.

14. The compound, according to claim 13, where R₉ is selected from among hydroxyl, -NR_{c}R_{d}, -NCOR_{c}, -CO₂R_{c}, -CONR_{c}R_{d}, -CN and -NO₂;
R_{c} and R_{d} are independently selected from among hydrogen, C₁-C₆ alkyl and C₃-C₇cycloalkyl; and
R_{c} and R_{d} can form a C₃-C₇carbocycle.

15. The compound, according to claim 13, which is selected from the list comprising:
1-benzyl-3-(2'-hydroxy-[1,1'-biphenyl]-4-yl)-1H-indole-2-carboxylic acid (7)
3-(2'-hydroxy-[1,1'-biphenyl]-4-il)-1H-indole-2-carboxylic acid (16).

16. A pharmaceutical composition comprising a compound, as defined in any of claims 10 to 15, or a pharmaceutically acceptable salt thereof, and at least one pharmaceutically acceptable adjuvant, carrier or excipient.

17. A use of a compound, as defined in any of claims 10 to 15, to manufacture a medicament.
